# EUROPEAN PATENT APPLICATION

(11) **EP 2 228 012 A1**
(43) Date of publication of application: **15.09.2010**
(21) Application number: 09172490.6
(22) Date of filing: 08.10.2009
(51) Int. Cl.: A61B 8/00

(54) **Probe for ultrasonic diagnosis apparatus**

(30) Priority: 12.03.2009 KR 20090021029
(71) Applicant: MEDISON CO., LTD., Gangwon-do 250-875 (KR)
(72) Inventor: Cho, Joo Yeon, 200-180, Gangwon-do (KR)
(74) Representative: Schmid, Wolfgang

(57) **Abstract**

Provided is a probe for an ultrasonic diagnostic apparatus. When an ultrasonic diagnosis is performed, a bubble removal member may collect bubbles within a liquid medium filled in the probe, and may discharge the collected bubbles to a region where a transfer of ultrasonic waves is unobstructed. Specifically, since the bubbles within the liquid medium do not obstruct the transfer of the ultrasonic waves, it is possible to enhance a probe performance. Also, it is possible to effectively remove the bubbles existing between a cover and a transducer or between the cover and a holder.

## Description

### BACKGROUND

### 1. Field of the Invention

The present invention relates to a probe used for an ultrasonic diagnostic apparatus, and more particularly, to a probe for an ultrasonic diagnostic apparatus that may fill a liquid medium within a probe and transfer ultrasonic waves using the liquid medium.

### 2. Description of the Related Art

Generally, an ultrasonic diagnostic apparatus may transmit ultrasonic waves towards a subject, and receive the ultrasonic waves reflected from the subject to thereby realize an internal shape of the subject. Since the ultrasonic waves have a different reflexibility in a boundary surface of two different materials, it is possible to obtain the internal shape of the subject by analyzing the reflected ultrasonic waves.

The ultrasonic diagnostic apparatus may include a probe to transmit and receive the ultrasonic waves to and from the subject. Specifically, the probe may transmit the ultrasonic waves to an inside of the subject, and may also receive the ultrasonic waves reflected from an internal tissue of the subject. A sectional image of a portion where the ultrasonic waves pass through may be created by reconstructing information received by the probe. The sectional image may be output on a monitor of the ultrasonic diagnostic apparatus. Through a review on the output image, a user may verify a size of the internal tissue of the subject, a shape thereof, a location thereof, a color thereof, and the like, and thereby may diagnose an internal status of the subject.

The probe may be classified into a linear type, a convex type, a circular type, and the like according to a type of a transducer. The probe may also be classified into the probe for a two-dimensional (2D) sectional image, the probe for a three-dimensional (3D) image, and the like according to the output image.

A conventional probe includes a transducer to transmit and receive ultrasonic waves, a contact portion being provided in front of the transducer to contact with a subject, and a liquid medium being filled in a space between the transducer and the contact portion to transfer the ultrasonic waves of the transducer to the contact portion. The liquid medium may contain bubbles. Also, due to an unskillfulness of an operator, or due to a complex internal structure of the probe, the bubbles may occur while filling or injecting the liquid medium.

When the bubbles are contained in the liquid medium of the probe, they may obstruct a transfer of the ultrasonic waves via the liquid medium. Accordingly, the ultrasonic waves may not be normally transferred from the transducer to the contact portion, which may result in deteriorating a probe performance and causing an inaccurate diagnosis.

### SUMMARY

An aspect of the present invention provides a probe for an ultrasonic diagnostic apparatus that may prevent a probe performance from being deteriorated due to bubbles within a liquid medium.

Another aspect of the present invention also provides a probe for an ultrasonic diagnostic apparatus that may guide bubbles within a liquid medium towards a region where a transfer of ultrasonic waves is unobstructed, using a simple structure and inexpensive costs.

According to an aspect of the present invention, there is provided a probe for an ultrasonic diagnostic apparatus, the probe including: a housing having an opening; a transducer being disposed in the opening of the housing to be partially exposed, and to transmit and receive ultrasonic waves when an ultrasonic diagnosis is performed; a holder being disposed in the opening of the housing to support the transducer; a cover being disposed to cover the opening of the housing to form a sealed space in the housing; a liquid medium being filled in the sealed space to transfer the ultrasonic waves of the transducer to the cover; and a bubble removal member being provided on the holder to collect bubbles within the liquid medium existing between the cover and the transducer or between the cover and the holder, and to discharge the bubbles to a region where a transfer of the ultrasonic waves is unobstructed.

When the bubble removal member discharges the bubbles to the region where the transfer of the ultrasonic waves are unobstructed, no bubble may exist in a transferring path of the ultrasonic waves transmitted to and received from the transducer. Therefore, it is possible to prevent an obstruction against the transfer of the ultrasonic waves that may be caused by the bubbles. Also, it is possible to prevent a performance of the probe from being deteriorated due to the bubbles within the liquid medium.

The transducer and the holder may be convexly provided in a form of a dome. The cover may be convexly provided in a form of a dome corresponding to the transducer and the holder. Specifically, since the transducer and the holder form an inclined curved shape starting from a center towards an edge, the bubbles within the liquid medium may smoothly move along the inclined curved shape according to a usage angle of the probe. Due to a weight difference between the bubbles and the liquid medium, or due to a vibration of the transducer, the bubbles may move into an opposite direction to a gravity.

The holder may be provided on each of both sides of the transducer. Therefore, a central portion of the transducer may be further protruded than the holder and the bubble removal member in the opening of the housing. The transducer may be provided between the holders, and thereby be vibratably supported by the holders.

A discharging path may be provided between the housing and the holder to discharge the bubbles within the liquid medium to an inside of the housing. When a location of the probe is changed into a direction where the cover is downwardly located, the bubbles existing between the cover and the transducer or between the cover and the holder may move to the opening of the housing along an inclined surface of the transducer and the holder. The bubbles moved to the opening of the housing may be discharged to the inside of the housing via the discharging path. Here, the inside of the housing corresponds to the region where the transfer of the ultrasonic waves transmitted to and received from the transducer is unobstructed.

An outlet may be formed in an opposite portion to the opening of the housing to discharge the bubbles existing in the housing to an outside of the housing. Specifically, the bubbles guided into the housing may be completely discharged to the outside of the housing via the outlet that is formed to face the opening.

The bubble removal member may include: a bubble collector being provided on the holder to collect the bubbles existing between the cover and the transducer or between the cover and the holder; and a bubble discharger being provided to pass through the holder and to discharge the collected bubbles to an inside of the housing. Specifically, the bubble collector may collect the bubbles within the liquid medium existing between the cover and the transducer to the bubble discharger. The bubble discharger may discharge the collected bubbles to the region where the transfer of the ultrasonic waves via the liquid medium is unobstructed. As described above, the inside of the housing may be the region where the transfer of the ultrasonic waves is unobstructed. However, the present invention is not limited thereto. As far as not obstructing the transfer of the ultrasonic waves, various types of portions may be used. For example, the bubbles discharged via the bubble discharger may be directly discharged to the outside of the housing, or may be received in a reception portion separately provided in the probe.

The bubble discharger may be provided as a bubble discharging path that is provided to pass through the holder from one side of the holder facing the cover to another side of the holder disposed in the housing. The bubble discharger may correspond to a path to discharge the bubbles existing between the cover and the transducer or between the cover and the holder to the inside of the housing. The bubble discharging path may be provided in a form of a hole formed in the holder or a pipe passing through the holder.

The bubble collector may include: a first guide protrusion guiding the bubbles within the liquid medium existing between the cover and the transducer or between the cover and the holder towards the opening of the housing; and a second guide protrusion guiding the bubbles within the liquid medium existing in the opening of the housing to the bubble discharger. Here, the first guide protrusion may be formed on the holder with an inlet of the bubble discharger to separate the inlet of the bubble discharger and the transducer. Also, the second guide protrusion may be formed on the holder and become wider from an inlet of the bubble discharger towards the opening of the housing.

When the usage angle of the probe is adjusted in a status where the cover downwardly faces, the bubbles within the liquid medium may upwardly move to the opening of the housing along the first guide protrusion due to a weight difference between the liquid medium and the bubbles, or due to a vibration of the transducer. The bubbles moved to the opening of the housing may move to the inside of the housing via the discharging path.

However, an interval between the cover and the holder or between the cover and the transducer may be narrow. Therefore, due to a surface tension, some of the bubbles may not upwardly move to the opening of the housing. Specifically, due to the surface tension occurring while the bubbles are moving to the opening of the housing, some of the bubbles may be suspended and may be in a stationary status for a long period of time.

When the angle of the probe is adjusted in a status where the cover upwardly faces or in a status where the cover is tilted at a predetermined angle, a direction of a gravity working on some of the bubbles may change into an opposite direction. Accordingly, some of the bubbles may upwardly move towards the transducer. Bubbles moved to the transducer may be collected in an inlet of the bubble discharger along the second guide protrusion.

The first guide protrusion and the second guide protrusion may be integrally formed with each other. The bubble collector may separate the inlet of the bubble discharger and the transducer, and may be provided in a parabolic form so that both ends of the bubble collector may become wider towards the opening of the housing.

Specifically, the bubble collector may be provided in a form of a U-shaped parabola that surrounds the inlet of the bubble discharger. The bubble collector may be provided in a form of a protruded rib or blade on the holder. Also, both ends of the bubble collector may be provided in separating directions. The first guide protrusion may correspond to a side of the bubble collector that faces the inlet of the bubble discharger. The second guide protrusion may correspond to another side of the bubble collector that does not face the inlet of the bubble collector.

### BRIEF DESCRIPTION OF THE DRAWINGS

These and/or other aspects, features, and advantages of the invention will become apparent and more readily appreciated from the following description of exemplary embodiments, taken in conjunction with the accompanying drawings of which:
- FIG. 1: is a perspective view illustrating an ultrasonic diagnostic apparatus including a probe according to an embodiment of the present invention;
- FIG. 2: is a perspective view illustrating main components of the probe of FIG. 1;
- FIG. 3: is a perspective view illustrating a status where a location of the probe of FIG. 2 is changed;
- FIG. 4: is a cross-sectional view illustrating main components of the probe of FIG. 3; and
- FIG. 5: is a cross-sectional view illustrating a bubble removal member of FIG 2.

### DETAILED DESCRIPTION

Reference will now be made in detail to exemplary embodiments of the present invention, examples of which are illustrated in the accompanying drawings, wherein like reference numerals refer to the like elements throughout. Exemplary embodiments are described below to explain the present invention by referring to the figures.

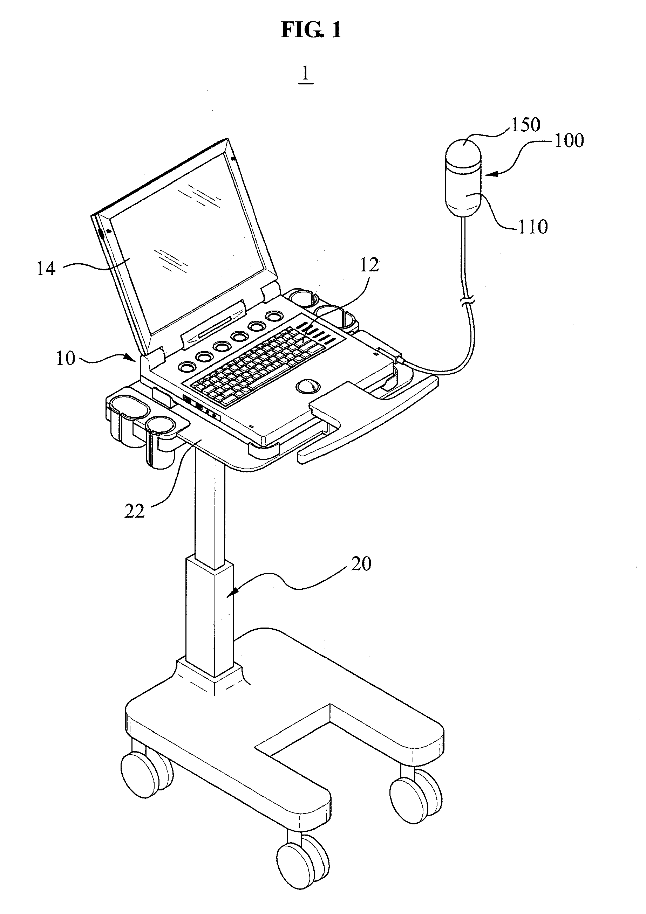

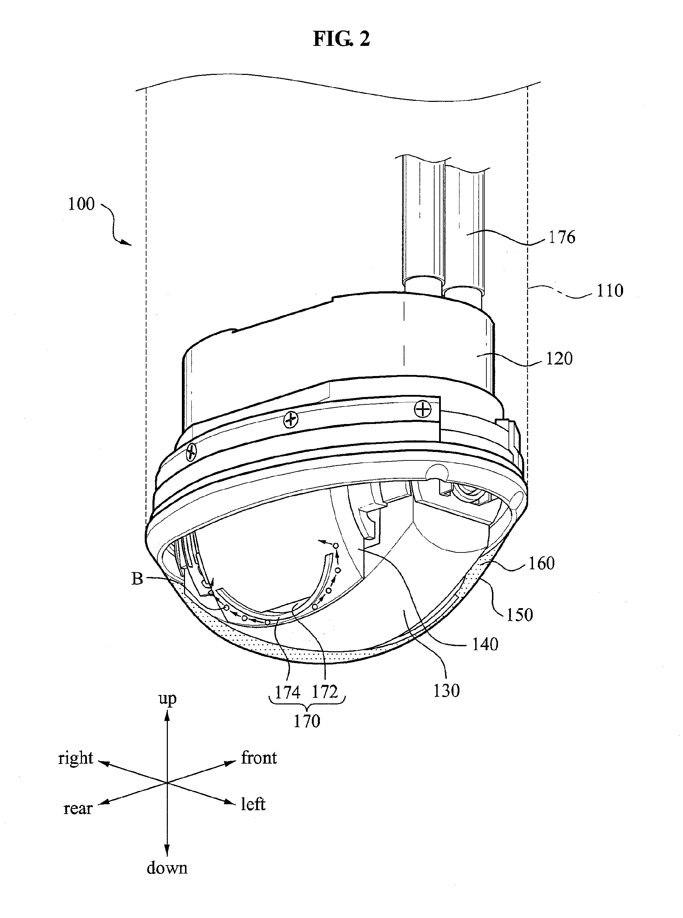

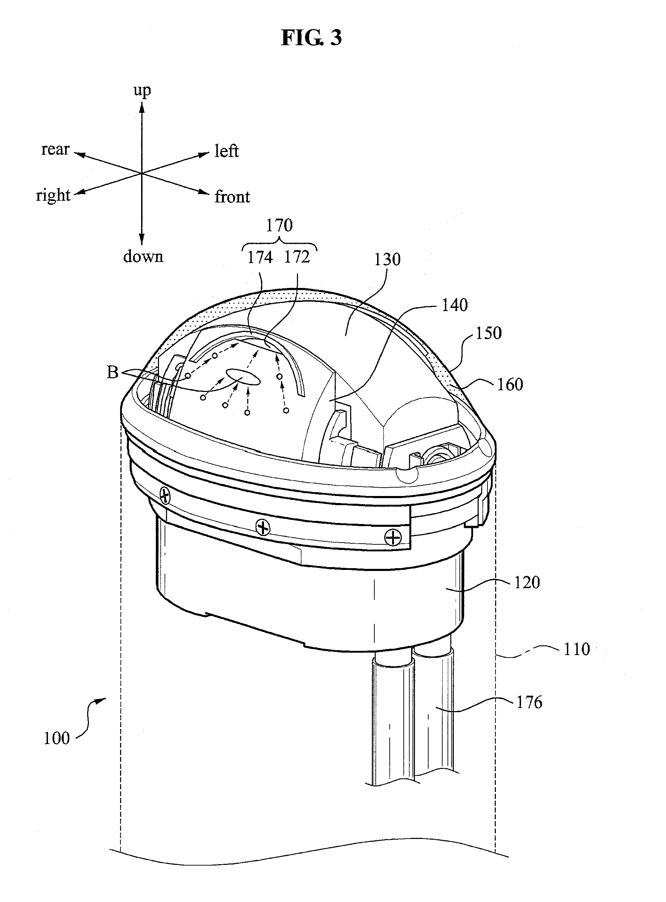

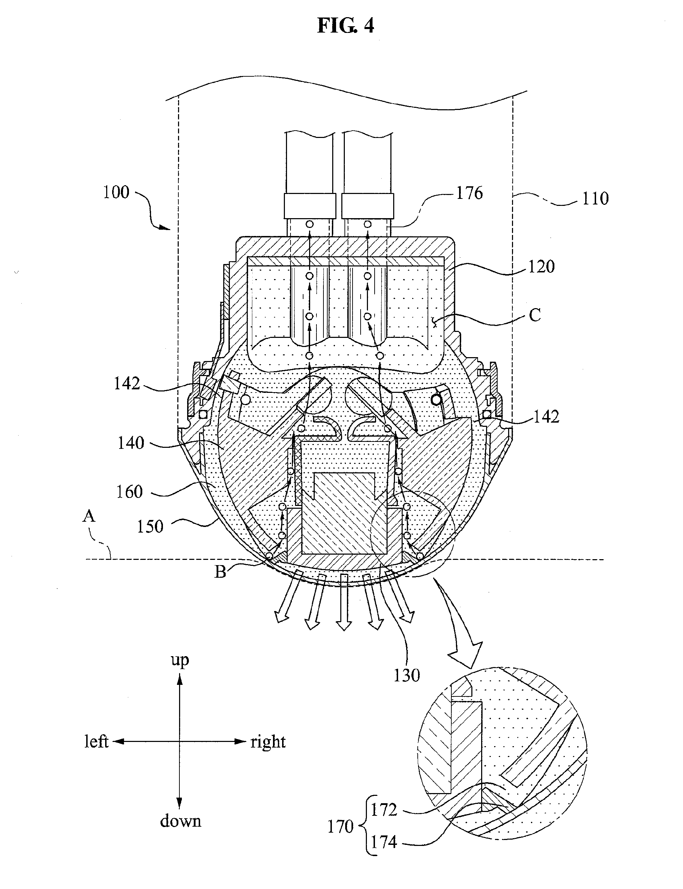

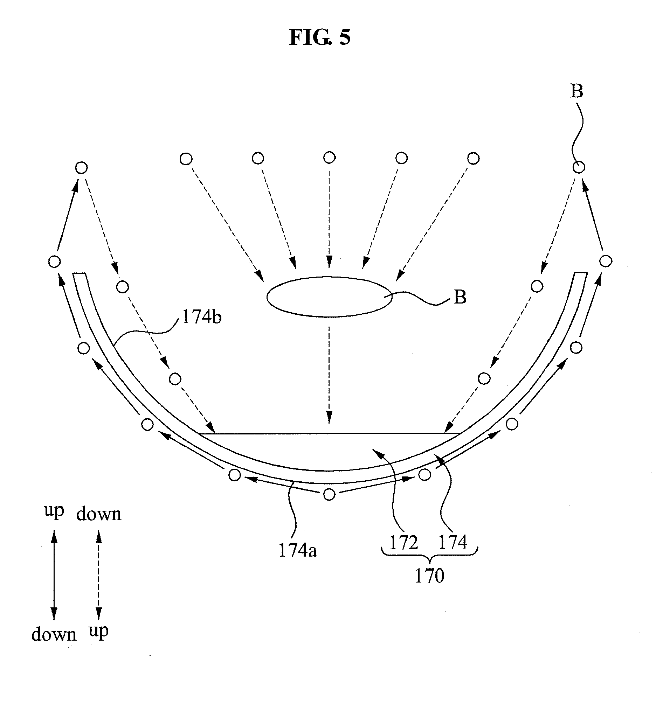

FIG. 1 is a perspective view illustrating an ultrasonic diagnostic apparatus 1 including a probe 100 according to an embodiment of the present invention, FIG. 2 is a perspective view illustrating main components of the probe 100 of FIG. 1, FIG. 3 is a perspective view illustrating a status where a location of the probe 100 of FIG. 2 is changed, FIG. 4 is a cross-sectional view illustrating main components of the probe 100 of FIG. 3, and FIG. 5 is a cross-sectional view illustrating a bubble removal member 170 of FIG. 2.

Referring to FIG. 1, the ultrasonic diagnostic apparatus 1 may include a main body 10 and a cart 20 including a desk 22 on which the main body 10 is disposed.

The main body 10 may include an operation portion 12 to input various types of commands and information required for an ultrasonic diagnosis, a display portion 14 to display an image when the ultrasonic diagnosis is performed, and the probe 100 being attached onto a subject to transmit ultrasonic waves to an inside of the subject and to receive the ultrasonic waves reflected from the subject. Hereinafter, the present invention will be described based on a case where the subject is a human being and the ultrasonic diagnostic apparatus 1 is used for medical purposes.

Referring to FIGS. 1 through 4, the probe 100 according to an embodiment of the present invention may include an external case 110, a housing 120, a transducer 130, a holder 140, a cover 150, a liquid medium 160, and the bubble removal member 170.

The external case 110 may form an external appearance of the probe 100. A radio communication apparatus or a cable connected to the main body 10 in a wired or wireless manner may be provided in the external case 110. Hereinafter, the present invention will be described based on a case where the probe 100 is connected to the main body 10 using the cable.

The housing 120 may be provided in a cylindrical form that has an opening on its one end. The housing 120 may be provided within the external case 110. The housing 120 may include the transducer 130, the holder 140, the liquid medium 160, and the bubble removal member 170.

For ease of description, in the present embodiment, directions, for example, up and down, front and rear, and left and right are set based on that the probe 100 is attached onto the subject A, as shown in FIGS. 2 and 4. Referring to FIGS. 2 and 4, the opening may be formed in a lower portion of the housing 120. An inside of the housing 120 may be positioned above the holder 140. Ultrasonic waves from the transducer 130 may be downwardly transferred.

The transducer 130 functions to transmit and receive the ultrasonic waves when the ultrasonic diagnosis is performed using the ultrasonic diagnostic apparatus 1. The transducer 130 may be provided in the middle of the opening of the housing 120. A lower portion of the transducer 130 may be exposed towards a lower portion of the opening of the housing 120.

The holder 140 may vibratably support the transducer 130. The holder 140 may be provided in the housing 120. The holder 140 may be provided in each of both sides of the transducer 130 in order to support the transducer 130 to be vibratable into front and rear direction. However, the holder 140 may support the transducer 130 to be vibratable into a direction different from the front and the rear directions.

The holder 140 and the transducer 130 may be convexly provided in a form of a dome in the opening of the housing 120. Accordingly, bubbles B within the liquid medium 160 may smoothly move into upward and downward directions along the surface of the transducer 130 and the holder 140.

A discharging path 142 may be provided between the housing 120 and the holder 140 to guide the bubbles B within the liquid medium 160 to the inside of the housing 120. Specifically, the bubbles B within the liquid medium 160 may exist between the transducer 130 and the cover 150 or between the holder 140 and the cover 150. When the probe 100 operates, the bubbles B may upwardly move along a bottom surface of the holder 140 and the transducer 130 due to a weight difference between the liquid medium 160 and the bubbles B, or due to the vibration of the transducer 130. The moved bubbles B may be discharged to the inside of the housing 120 via the discharging path 142. Here, the inside of the housing may be a region where a transfer of the ultrasonic waves via the liquid medium 160 is unobstructed.

The cover150 may transfer the ultrasonic waves between the subject A and the liquid medium 160. When the ultrasonic diagnosis is performed using the ultrasonic diagnostic apparatus 1, the cover 150 may contact with the subject A. The cover 150 may be tightly coupled with the opening of the housing 120. Through this, a sealed space C may be formed in a space between the housing 120 and the cover 150 to be filled with the liquid medium 160. The cover 150 may be convexly provided in a form of a dome corresponding to the transducer 130 and the holder 140.

The liquid medium 160 may be a flowing medium material that may transfer the ultrasonic waves of the transducer 130 to the cover 150. The liquid medium 160 may be filled in the sealed space C formed between the housing 120 and the cover 150. An oil or a saline solution may be used for the liquid medium 160. Hereinafter, the present invention will be described based on a case where the oil is used for the liquid medium 160.

The bubble removal member 170 may collect the bubbles B within the liquid medium 160 existing between the transducer 130 and the cover 150, and may guide the collected bubbles B to the region where the transfer of the ultrasonic waves is unobstructed. The bubble removal member 170 may be disposed in a location above a lowest portion of the bottom surface of the transducer 130 contacting with the liquid medium 160. This is because, when the probe 100 is attached onto the subject A, the bubbles B within the liquid medium 160 existing between the transducer 130 and the cover 150 may move to the bubble removal member 170 along the bottom surface of the transducer 130 and the holder 140.

The region where the transfer of the ultrasonic waves is unobstructed may include all the internal space of the probe 100 excluding a space formed between the transducer 130 and the cover 150. Specifically, the region may correspond to portions excluding a path of the transducer 130 used for transmitting and receiving the ultrasonic waves. Hereinafter, the present invention will be described based on a case where the region includes an upper portion of the holder 140, that is, the inside of the housing 120. Accordingly, the bubble removal member 170 may discharge the bubbles B within the liquid medium 160, existing between the transducer 130 and the cover 150, to the inside of the housing 120. Through this, it is possible to remove all the bubbles B existing between the transducer 130 and the cover 150, and to prevent the bubbles B from obstructing the transfer of the ultrasonic waves.

As shown in FIGS. 2 through 5, the bubble removal member 170 may include a bubble discharger 172 and a bubble collector 174.

The bubble discharger 172 may discharge the bubbles B, collected by the bubble collector 174, to the inside of the housing 120. The bubble discharger 172 may be formed to pass through the holder 140. For example, the bubble discharger 172 may be provided as a bubble discharge path that is formed to pass through the holder 140 from the bottom surface of the holder 140 to the top surface of the holder 140.

The bubble discharger 172 may be formed in the holder 140 in various types of paths and in various types of forms. Hereinafter, the present invention will be described based on a case where the bubble discharger 172 is provided in a form of a hole in the holder 140. However, the bubble discharger 172 may be provided in a form of a pipe that passes through the holder 140.

The bubble discharger 172 may be provided in a location above a lowest portion of the transducer 130. Through this, it is possible to secure a space where the bubble collector 174 may further collect the bubbles B existing in the upper portion of the holder 140.

The bubble collector 174 may collect, towards the bubble discharger 172, the bubbles B within the liquid medium 160 existing between the transducer 130 and the cover 150 or between the holder 140 and the cover 150. The bubble collector 174 may be provided in a protruded form on the holder 140.

For example, the bubble collector 174 may include a first guide protrusion 174a and a second guide protrusion 174b. The first guide protrusion 174a may be provided around an inlet of the bubble discharger 172 to guide the bubbles B within the liquid medium 160 towards the opening of the housing 120. The second guide protrusion 174b may become wider from the inlet of the bubble discharger 172 towards the opening of the housing 120, and may guide the bubbles B existing in the opening of the housing 120 to the bubble discharger 172. The first guide protrusion 174a and the second guide protrusion 174b may be provided in a form of a protruded rib on the bottom surface of the holder 140.

When the probe 100 is used as shown in FIGS. 2 and 4, the first guide protrusion 174a may guide the bubbles B existing between the transducer 130 and the cover 150 or between the holder 140 and the cover 150 to move towards the opening of the housing 120. Here, due to the weight difference between the bubbles B and the liquid medium 160, or due to the vibration of the transducer 130, the bubbles B may move long the bottom surface of the transducer 130 and the holder 140.

The first guide protrusion 174a may be provided between the transducer 130 and the inlet of the bubble discharger 172. Accordingly, the first guide protrusion 174 may prevent the bubbles B moving towards the upper portion of the holder 140 from moving to the inlet of the bubble discharger 172.

When the probe 100 is used as shown in FIG. 3, the second guide protrusion 174b may move the bubbles B existing between the holder 140 and the cover 150 or between the inlet of the housing 120 and the holder 140 to the bubble discharger 172. Here, due to the weight difference between the bubbles B and the liquid medium 160, or due to the vibration of the transducer 130, the bubbles B may move long the bottom surface of the transducer 130 and the holder 140.

The second guide protrusion 174b may be provided in a structure to become wider from the inlet of the bubble discharger 172 towards the opening of the housing 120. Accordingly, the second guide protrusion 174b may collect the bubbles B moving along the holder 140, in the inlet of the bubble discharger 172.

Depending on embodiments, the first guide protrusion 174a and the second guide protrusion 174b may be separately provided. However, in the present embodiment, the first guide protrusion 174a and the second guide protrusion 174b are integrally provided. Specifically, the bubble collector 174 may separate the transducer 130 and the inlet of the bubble discharger 172. The bubble collector 174 may be provided in a parabolic form so that both ends of the bubble collector 174 may become wider towards the opening of the housing 120. The bubble collector 174 may be disposed in a portion corresponding to a displacement point of a parabola between the transducer 130 and the inlet f the bubble discharger 172, and may be in a concave parabolic form with respect to the bubble discharger 172.

An outlet 176 may be provided in the upper portion of the housing 120 to discharge the bubbles B existing in the housing 120 to an outside of the housing 120. Specifically, when the bubbles B are guided into the housing 120 via the discharging path 142 or the bubble discharger 172, the bubbles B may be discharged to the outside of the housing 120 via the outlet 176 that is provided in an opposite location to the opening of the housing 120.

Hereinafter, an operation of the probe 100 for the ultrasonic diagnostic apparatus 1 constructed as above according to an embodiment of the present invention will be described.

When an ultrasonic diagnosis is performed using the ultrasonic diagnostic apparatus 1, the cover 150 of the probe 100 may contact with the subject A. The transducer 130 may be operated to transmit and receive ultrasonic waves to and from the subject A.

The ultrasonic waves transmitted to the transducer 130 may be transferred to the cover 150 using the liquid medium 160. The transferred ultrasonic waves may be transferred to the subject A via the cover 150. The ultrasonic waves reflected from the subject A may be transferred to the transducer 130 using the cover 150 and the liquid medium 160. An image associated with an internal tissue of the subject A may be constructed by analyzing the ultrasonic waves transferred to the transducer 130.

Referring to FIGS. 2, 3, and 5, when the ultrasonic diagnosis is performed, the probe 100 may move in a status of contacting with the subject A. Hereinafter, the present invention will be described, assuming that the subject A exists below the probe 100 and the bubbles B exist in the liquid medium 160.

Accordingly, the bubbles B may be contained in the liquid medium 160 existing between the transducer 130 and the cover 150 or between the holder 140 and the cover 150. Due to a weight difference with the liquid medium 160, or due to a vibration of the transducer 130, the bubbles B may upwardly move along the transducer 130 and the holder 140.

In FIGS. 2, 4, and 5, a path where the bubbles B move to the upper portion of the holder 140 is indicated by solid arrow indicators. Specifically, the bubbles B within the liquid medium 160 may move to the upper portion of the holder 140 along one convex side of the bubble collector 174. The bubbles B guided between the upper portion of the holder 140 and the opening of the housing 110 may move to the inside of the housing 120 via the discharging path 142. Here, some of the bubbles B moved to the upper portion of the holder 140 may remain between the upper portion of the holder 140 and the cover 150, without moving to the discharging path 142, and may be in a stationary status for a long period of time.

Referring to FIGS. 3 and 5, the probe 100 is provided in an opposite direction to an operation direction of the probe 100 of FIGS. 2 and 4. When the ultrasonic diagnosis is completed or is temporarily suspended, the probe 100 may be maintained in the main body 10 or the cart 20 in the opposite direction to the operation direction of the probe 100 of FIGS. 2 and 4. Also, when the ultrasonic diagnosis is performed using the ultrasonic diagnostic apparatus 1, the probe 100 may be used at various angles or in various directions. For example, the probe 100 may be used in the opposite direction to the operation direction of FIGS. 2 and 4, or may be used in a titled status at a predetermined angle.

When the direction of the probe 100 is changed, the bubbles B stationary between the upper portion of the holder 140 and the cover 150 may upwardly move towards the transducer 130. In FIGS. 3 and 5, a path where the bubbles B upwardly move towards the transducer 130 is indicated by dotted arrow indicators.

Specifically, the bubbles B stationary between the holder 140 and the cover 150 may upwardly move along the surface of the holder 140, and may be collected in the bubble discharger 172 along concave another surface of the bubble collector 174. The bubbles B stationary between the upper portion of the holder 140 and the cover 150 may move to the bubble discharger 172.

Next, when the direction of the probe 100 is changed into the direction as shown in FIGS. 2 and 4, the bubbles B may move to the inside of the housing 120 along the bubble discharger 172. The bubbles B moved to the inside of the housing 120 may be completely discharged to the outside of the housing 120 via the outlet 176.

According to an embodiment of the present invention, there is provided a probe for an ultrasonic diagnostic apparatus that may move, using a bubble removal member, bubbles existing between a transducer and a cover to a region where a transfer of ultrasonic waves is unobstructed. Therefore, the probe may smoothly transfer the ultrasonic waves using a liquid medium without being obstructed by the bubbles.

Also, according to an embodiment of the present invention, there is provided a probe for an ultrasonic diagnostic apparatus that may prevent a probe performance from being deteriorated due to bubbles within a liquid medium. Therefore, when a diagnosis is performed using the ultrasonic diagnostic apparatus, it is possible to obtain more accurate information.

Also, according to an embodiment of the present invention, there is provided a probe for an ultrasonic diagnostic apparatus that may smoothly move bubbles within a liquid medium to a bubble removal member or a discharging path along a holder and a transducer that are provided in a form of a dome, and may effectively discharge the bubbles within the liquid medium to an inside of a housing where a transfer of ultrasonic waves is unobstructed by the bubble removal member or the discharging path.

Also, according to an embodiment of the present invention, there is provided a probe for an ultrasonic diagnostic apparatus that may collect, using a bubble collector, bubbles existing between a cover and a transducer, and may discharge, using a bubble discharger, the collected bubbles to an inside of a housing. Therefore, it is possible to prevent the bubbles from being in a stationary status for a long period of time, and to significantly reduce a number of operations that are performed to remove the bubbles.

Also, according to an embodiment of the present invention, there is provided a probe for an ultrasonic diagnostic apparatus that may simply remove bubbles existing between a cover and a transducer. Therefore, it is possible to construct various types of probes with inexpensive costs.

Although a few exemplary embodiments of the present invention have been shown and described, the present invention is not limited to the described exemplary embodiments. Instead, it would be appreciated by those skilled in the art that changes may be made to these exemplary embodiments without departing from the principles and spirit of the invention, the scope of which is defined by the claims and their equivalents.

## Claims

1. A probe for an ultrasonic diagnostic apparatus, the probe comprising:
a housing having an opening;
a transducer being disposed in the opening of the housing to be partially exposed, and to transmit and receive ultrasonic waves when an ultrasonic diagnosis is performed;
a holder being disposed in the opening of the housing to support the transducer;
a cover being disposed to cover the opening of the housing to form a sealed space in the housing;
a liquid medium being filled in the sealed space to transfer the ultrasonic waves of the transducer to the cover; and
a bubble removal member being provided on the holder to collect bubbles within the liquid medium existing between the cover and the transducer or between the cover and the holder, and to discharge the bubbles to a region where a transfer of the ultrasonic waves is unobstructed.

2. The probe of claim 1, wherein:
the transducer and the holder are convexly provided in a form of a dome, and
the cover is convexly provided in a form of a dome corresponding to the transducer and the holder, and
the holder is provided on each of both sides of the transducer.

3. The probe of claim 2, wherein a discharging path is formed between the housing and the holder to discharge the bubbles within the liquid medium to an inside of the housing.

4. The probe according to any one of claims 1 through 3, wherein the bubble removal member comprises:
a bubble collector being provided on the holder to collect the bubbles existing between the cover and the transducer or between the cover and the holder; and
a bubble discharger being provided to pass through the holder, and to discharge the collected bubbles to the inside of the housing.

5. The probe of claim 4, wherein the bubble discharger is provided as a bubble discharging path that is provided to pass through the holder from one side of the holder facing the cover to another side of the holder disposed in the housing.

6. The probe of claim 5, wherein an outlet is formed in an opposite portion to the opening of the housing to discharge the bubbles existing in the housing to an outside of the housing.

7. The probe of claim 5, wherein the bubble collector comprises:
a first guide protrusion being provided between an inlet of the bubble discharger and the transducer to guide the bubbles within the liquid medium existing between the cover and the transducer or between the cover and the holder towards the opening of the housing; and
a second guide protrusion being provided in a form that becomes wider from the inlet of the bubble discharger towards the opening of the housing to guide the bubbles within the liquid medium existing in the opening of the housing to the bubble discharger.

8. The probe of claim 5, wherein the bubble collector separates an inlet of the bubble discharger and the transducer, and is provided in a parabolic form so that both ends of the bubble collector become wider towards the opening of the housing.
